(19) Europäisches Patentamt
European Patent Office
Office européen des brevets

(11) EP 3 941 541 B1

(12) # EUROPEAN PATENT SPECIFICATION

(45) Date of publication and mention of the grant of the patent:
**20.03.2024 Bulletin 2024/12**

(21) Application number: **20788692.0**

(22) Date of filing: **13.04.2020**

(51) International Patent Classification (IPC):
***A61L 15/60*** *(2006.01)* ***A61K 47/32*** *(2006.01)*
***A61K 47/34*** *(2017.01)* ***A61L 15/42*** *(2006.01)*
***A61L 26/00*** *(2006.01)*

(52) Cooperative Patent Classification (CPC):
(C-Sets available)
**C08J 9/0061; A61L 26/0052; A61L 26/0066; A61L 26/008; C08J 9/08; C08L 29/04;**
A61L 2300/222; A61L 2300/402; A61L 2300/41; C08J 2203/02; C08J 2205/022; C08J 2207/12; C08J 2329/04; C08J 2401/26; C08J 2435/08;

(Cont.)

(86) International application number:
**PCT/US2020/027965**

(87) International publication number:
**WO 2020/210815 (15.10.2020 Gazette 2020/42)**

(54) **SUPERPOROUS HYDROGELS, METHODS OF MAKING THE SAME, AND ARTICLES INCORPORATING THE SAME**

SUPERPORÖSE HYDROGELE, VERFAHREN ZU DEREN HERSTELLUNG UND DIESE ENTHALTENDE ARTIKEL

HYDROGELS SUPERPOREUX, LEURS PROCÉDÉS DE FABRICATION ET ARTICLES LES INCORPORANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.04.2019 US 201962832777 P**

(43) Date of publication of application:
**26.01.2022 Bulletin 2022/04**

(73) Proprietor: **ConvaTec Technologies Inc.**
**Las Vegas NV 89169 (US)**

(72) Inventors:
- **CALO, Enrica**
  **Reading RG6 6AD (GB)**
- **BALLAMY, Lucy**
  **Deeside CH5 2NU (GB)**
- **KHUTORYANSKIY, Vitaliy, V.**
  **Reading RG6 6AD (GB)**

(74) Representative: **Wilson Gunn**
**Blackfriars House**
**The Parsonage**
**5th Floor**
**Manchester M3 2JA (GB)**

(56) References cited:
**US-A- 5 405 366 US-A1- 2011 160 398**

- **TANG CHUNYI ET AL: "Electrospinning of poly(styrene-co-maleic anhydride) (SMA) and water-swelling behavior of crosslinked/hydrolyzed SMA hydrogel nanofibers", POLYMER, vol. 48, no. 15, 1 July 2007 (2007-07-01), pages 4482-4491, XP055908741, AMSTERDAM, NL ISSN: 0032-3861, DOI: 10.1016/j.polymer.2007.05.041**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CALO: "Developing new interface materials for wound care applications", Submitted as partial fulfilment for the degree of Doctor of Philosophy, October 2016 (2016-10), pages iii, 50 , 71 , 72 , 99-114, XP055748691, Retrieved from the Internet: URL:http://centaur.reading.ac.uk/72919/1/21820305_Calo_thesis.pdf [retrieved on 2020-05-28]**
- **DEMIR et al.: "Poly (methyl vinyl ether-co-maleic acid) - Pectin based hydrogel-forming systems: Gel, film, and microneedles", European Journal of Pharmaceutics and Biopharmaceutics, vol. 117 21 April 2017 (2017-04-21), pages 182-194, XP055491789, DOI: 10.1016/j.ejpb.2017.04.018 Retrieved from the Internet: URL:https://www.sciencedirect.com/science/article/abs/pii/S0939641117301133?via%3Dihub [retrieved on 2020-05-28]**
- **Li Yang ET AL: "Construction of porous sponge-like PVA-CMC-PEG hydrogels with pH-sensitivity via phase separation for wound dressing", International Journal of Polymeric Materials and Polymeric Biomaterials, vol. 69, no. 8, 7 March 2019 (2019-03-07), pages 505-515, XP093036215, US ISSN: 0091-4037, DOI: 10.1080/00914037.2019.1581200**



(52) Cooperative Patent Classification (CPC): (Cont.)
C08L 1/284; C08L 25/08

C-Sets
**A61L 26/0052, C08L 1/28;**
**A61L 26/0052, C08L 29/04;**
**A61L 26/0052, C08L 33/06;**
**A61L 26/0052, C08L 35/08;**
**C08L 29/04, C08K 2003/262, C08L 35/08**

## Description

## CROSS-REFERENCE TO RELATED APPLICATION

This application claims priority to, and the benefit of, U.S. Provisional Patent Application Serial No. 62/832,777 entitled "Superporous Hydrogel Foams," which was filed on April 11, 2019.

## FIELD OF THE DISCLOSURE

The present disclosure relates, generally, to hydrogels, and, more specifically, to superporous hydrogels (SPHs) adapted for use in wound dressings.

## BACKGROUND

Hydrogels may be used for the design of many biomedical products. For example, hydrogels may be adapted for use in, or incorporated into, controlled-release drug delivery devices, engineered scaffolds for tissue or organ replacement, wound dressings, contact lenses, and personal hygiene items.

Superporous hydrogels (SPHs) are chemically crosslinked hydrophilic polymers that may contain a multiplicity of pores with diameters in the micrometer to millimeter range. The pores may be sized to enable the SPHs to absorb tens of times their weight of aqueous fluids in short amounts of time, such as within fractions of a minute, for example. SPH pores are typically interconnected in the hydrogel matrix to allow absorbing fluid to move freely through channels or capillaries formed by the matrix and/or the pores. As a result, the pores of SPHs facilitate, or are otherwise associated with, swelling at considerably faster rates than conventional hydrogels with comparable swelling capacities.

## SUMMARY

The present disclosure may comprise one or more of the following features and combinations thereof.

According to one aspect of the present disclosure, there is provided a wound dressing comprising a polymeric superporous hydrogel comprising a copolymer of poly(alkylvinylether/maleic acid), or a copolymer of poly(styrene/maleic anhydride). The polymeric superporous hydrogel includes polyvinyl alcohol (PVA) or hydroxyethylcellulose (HEC). The polymeric superporous hydrogel includes a plurality of pores.

In some embodiments, the copolymer of poly(alkylvinylether/maleic acid) may be poly(methyl vinyl ether-*alt*-maleic acid) (PMVEMAC).

In some embodiments, the copolymer of poly(styrene/maleic anhydride) may be poly(styrene-co-maleic anhydride) (SMA).

In some embodiments, the polymeric superporous hydrogel may include polyvinyl alcohol (PVA).

In some embodiments, an average pore size of the plurality of pores may range from about 5 μm to about 1 mm.

In some embodiments, the polymeric superporous hydrogel may exhibit a swelling ratio of between about 4000% and about 8500% after 24 hours.

In some embodiments, the polymeric superporous hydrogel may exhibit a swelling ratio of about 4000% after 24 hours.

In some embodiments, the polymeric superporous hydrogel may exhibit a swelling ratio of about 7000% after 24 hours.

In some embodiments, the polymeric superporous hydrogel may exhibit a swelling ratio of about 8500% after 24 hours.

In some embodiments, the polymeric superporous hydrogel may exhibit intrinsic antimicrobial activity.

In some embodiments, the polymeric superporous hydrogel may exhibit intrinsic antimicrobial activity against Gram-positive bacteria.

In some embodiments, the polymeric superporous hydrogel may exhibit intrinsic antimicrobial activity against *Staphylococcus aureus.*

In some embodiments, the polymeric superporous hydrogel may exhibit an elastic modulus (Young's modulus) between about $1\times10^{-5}$ N/mm$^2$ and about $4\times10^{-5}$ N/mm$^2$.

In some embodiments, the polymeric superporous hydrogel may exhibit an elastic modulus (Young's modulus) between about $1\times10^{-5}$ N/mm$^2$ and about $3\times10^{-5}$ N/mm$^2$.

In some embodiments, the polymeric superporous hydrogel may exhibit an elastic modulus (Young's modulus) between about $2.9\times10^{-5}$ N/mm$^2$ and about $3.7\times10^{-5}$ N/mm$^2$.

In some embodiments, the polymeric superporous hydrogel may exhibit an elastic modulus (Young's modulus) between about $1.4\times10^{-5}$ N/mm$^2$ and about $3.8\times10^{-5}$ N/mm$^2$.

In some embodiments, the polymeric superporous hydrogel may exhibit between about 44% and about 100% elongation at break.

In some embodiments, the polymeric superporous hydrogel may exhibit between about 44% and about 72% elongation at break.

In some embodiments, the polymeric superporous hydrogel may exhibit between about 80% and about 96% elongation at break.

In some embodiments, the polymeric superporous hydrogel may exhibit between about 69% and about 96% elongation at break.

In some embodiments, the polymeric superporous hydrogel may exhibit a tensile stress at break between about $0.3\times10^{-5}$ N/mm$^2$ and about $1.3\times10^{-4}$ N/mm$^2$.

In some embodiments, the polymeric superporous hydrogel may exhibit a tensile stress at break between about $2.1\times10^{-5}$ N/mm$^2$ and about $7.3\times10^{-5}$ N/mm$^2$.

In some embodiments, the polymeric superporous hydrogel may exhibit a tensile stress at break between about $0.7\times10^{-4}$ N/mm$^2$ and about $1.3\times10^{-4}$ N/mm$^2$.

In some embodiments, the polymeric superporous hydrogel may exhibit a tensile stress at break between about $0.3\times10^{-5}$ N/mm$^2$ and about $1.9\times10^{-5}$ N/mm$^2$.

In some embodiments, the polymeric superporous hydrogel may exhibit a maximum strength between about $3\times10^{-2}$ N/mm and about $6.3\times10^{-2}$ N/mm.

In some embodiments, the polymeric superporous hydrogel may exhibit a maximum strength between about $3\times10^{-2}$ N/mm and about $4.4\times10^{-2}$ N/mm.

In some embodiments, the polymeric superporous hydrogel may exhibit a maximum strength between about $3.8\times10^{-2}$ N/mm and about $5.2\times10^{-2}$ N/mm.

In some embodiments, the polymeric superporous hydrogel may exhibit a maximum strength between about $3.1\times10^{-2}$ N/mm and about $6.3\times10^{-2}$ N/mm.

In some embodiments, the polymeric superporous hydrogel may not contain any trace amount of initiators.

In some embodiments, the polymeric superporous hydrogel may not contain any trace amount of foam stabilizers.

In some embodiments, the polymeric superporous hydrogel may not contain any trace amount of organic solvents.

According to the present disclosure, a method of making a polymeric superporous hydrogel is disclosed, but not claimed, the method including (a) a copolymer of poly(alkylvinylether/maleic acid), or a copolymer of poly(styrene/maleic anhydride), (b) polyvinyl alcohol (PVA) or hydroxyethylcellulose (HEC), and (c) a plurality of pores may include (i) mixing an aqueous solution of a copolymer of poly(alkylvinylether/maleic acid), a copolymer of poly(alkylvinylether/maleic anhydride), or a copolymer of poly(styrene/maleic anhydride) with an aqueous solution of polyvinyl alcohol (PVA) or hydroxyethylcellulose (HEC), (ii) adding a blowing agent to form gas bubbles, and (iii) heating the resulting mixture at elevated pressure to form the polymeric superporous hydrogel. The embodiments relating to the method listed under are not claimed, thus, not part of the invention.

In some embodiments, the copolymer of poly(alkylvinylether/maleic acid) may be poly(methyl vinyl ether-*alt*-maleic acid) (PMVEMAC).

In some embodiments, the copolymer of poly(styrene/maleic anhydride) may be poly(styrene-co-maleic anhydride) (SMA).

In some embodiments, the polymeric superporous hydrogel may include polyvinyl alcohol (PVA).

In some embodiments, the blowing agent may be a carbonate.

In some embodiments, the blowing agent may be sodium carbonate, sodium bicarbonate, potassium carbonate, potassium bicarbonate, calcium carbonate, calcium bicarbonate, or lithium carbonate.

In some embodiments, the carbonate may be sodium bicarbonate.

In some embodiments, the blowing agent may be a solid substance.

In some embodiments, the amount of the blowing agent may be between about 0.1% w/v and about 1% w/v.

In some embodiments, the amount of the blowing agent may be about 0.4% w/v.

In some embodiments, the amount of the blowing agent may be about 0.5% w/v.

In some embodiments, the amount of the blowing agent may be about 0.7% w/v.

In some embodiments, heating the resulting mixture at elevated pressure may be conducted for between about 30 min and about 2 hr.

In some embodiments, heating the resulting mixture at elevated pressure may include heating the resulting mixture at between about 110 °C and about 140 °C.

In some embodiments, the elevated pressure may be between about 15 psi and about 40 psi.

In some embodiments, the method of making may not include using additional crosslinkers.

In some embodiments, the method of making may not include using initiators.

In some embodiments, the method of making may not include using foam stabilizers.

In some embodiments, the method of making may not include using organic solvents.

In some embodiments, the method of making may not include any purification steps.

In some embodiments, an average pore size in the polymeric superporous hydrogel may range from about 5 μm to about 1 mm.

In some embodiments, the polymeric superporous hydrogel may exhibit a swelling ratio between about 4000% and about 8500% after 24 hours.

In some embodiments, the polymeric superporous hydrogel may exhibit a swelling ratio of about 4000% after 24 hours.

In some embodiments, the polymeric superporous hydrogel may exhibit a swelling ratio of about 7000% after 24 hours.

In some embodiments, the polymeric superporous hydrogel may exhibit a swelling ratio of about 8500% after 24 hours.

In some embodiments, the polymeric superporous hydrogel may exhibit intrinsic antimicrobial activity.

In some embodiments, the polymeric superporous hydrogel may exhibit intrinsic antimicrobial activity against Gram-positive bacteria.

In some embodiments, the polymeric superporous hydrogel may exhibit intrinsic antimicrobial activity against *Staphylococcus aureus.*

In some embodiments, the polymeric superporous hydrogel may exhibit an elastic modulus (Young's modulus) between about $1\times10^{-5}$ N/mm$^2$ and about $4\times10^{-5}$ N/mm$^2$.

In some embodiments, the polymeric superporous hydrogel may exhibit an elastic modulus (Young's modulus) between about $1\times10^{-5}$ N/mm$^2$ and about $3\times10^{-5}$ N/mm$^2$.

In some embodiments, the polymeric superporous hydrogel may exhibit an elastic modulus (Young's modulus) between about $2.9\times10^{-5}$ N/mm$^2$ and about $3.7\times10^{-5}$ N/mm$^2$.

In some embodiments, the polymeric superporous hydrogel may exhibit an elastic modulus (Young's modulus) between about $1.4\times10^{-5}$ N/mm$^2$ and about $3.8\times10^{-5}$ N/mm$^2$.

In some embodiments, the polymeric superporous hydrogel may exhibit between about 44% and about 100% elongation at break.

In some embodiments, the polymeric superporous hydrogel may exhibit between about 44% and about 72% elongation at break.

In some embodiments, the polymeric superporous hydrogel may exhibit between about 80% and about 96% elongation at break.

In some embodiments, the polymeric superporous hydrogel may exhibit between about 69% and about 96% elongation at break.

In some embodiments, the polymeric superporous hydrogel may exhibit a tensile stress at break between about $0.3\times10^{-5}$ N/mm$^2$ and about $1.3\times10^{-4}$ N/mm$^2$.

In some embodiments, the polymeric superporous hydrogel may exhibit a tensile stress at break between about $2.1\times10^{-5}$ N/mm$^2$ and about $7.3\times10^{-5}$ N/mm$^2$.

In some embodiments, the polymeric superporous hydrogel may exhibit a tensile stress at break between about $0.7\times10^{-4}$ N/mm$^2$ and about $1.3\times10^{-4}$ N/mm$^2$.

In some embodiments, the polymeric superporous hydrogel may exhibit a tensile stress at break between about $0.3\times10^{-5}$ N/mm$^2$ and about $1.9\times10^{-5}$ N/mm$^2$.

In some embodiments, the polymeric superporous hydrogel may exhibit a maximum strength between about $3\times10^{-2}$ N/mm and about $6.3\times10^{-2}$ N/mm.

In some embodiments, the polymeric superporous hydrogel may exhibit a maximum strength between about $3\times10^{-2}$ N/mm and about $4.4\times10^{-2}$ N/mm.

In some embodiments, the polymeric superporous hydrogel may exhibit a maximum strength between about $3.8\times10^{-2}$ N/mm and about $5.2\times10^{-2}$ N/mm.

In some embodiments, the polymeric superporous hydrogel may exhibit a maximum strength between about $3.1\times10^{-2}$ N/mm and about $6.3\times10^{-2}$ N/mm.

According to the present disclosure, a method of making a polymeric superporous hydrogel is disclosed, but not claimed, the method including (a) a copolymer of poly(alkylvinylether/maleic acid), a copolymer of poly(alkylvinylether/maleic anhydride), or a copolymer of poly(styrene/maleic anhydride), (b) polyvinyl alcohol (PVA) or hydroxyethylcellulose (HEC), and (c) a plurality of pores may include (i) mixing an aqueous solution of a copolymer of poly(alkylvinylether/maleic acid) or a copolymer of poly(alkylvinylether/maleic anhydride) with an aqueous solution of polyvinyl alcohol (PVA), hydroxyethylcellulose (HEC), or poly (acrylic acid) (PAA), (ii) adding a blowing agent as a solid to form gas bubbles, and (iii) heating the resulting mixture at elevated pressure to form the polymeric superporous hydrogel.

According to yet another aspect of the present disclosure still, a wound dressing may incorporate a polymeric

superporous hydrogel as disclosed herein.

In some embodiments, the wound dressing may include an active agent.

In some embodiments, the wound dressing may include an active agent selected from anti- inflammatory agents, anesthetics, and combination thereof.

In some embodiments, the active agent may be a topical corticosteroid.

In some embodiments, the active agent may be hydrocortisone, clobetasone butyrate, or betamethasone dipropionate.

In some embodiments, the active agent may be lidocaine or benzocaine.

According to a further aspect of the present disclosure, a polymeric superporous hydrogel may be obtained by a method disclosed herein.

According to the present disclosure still, a method of treating Gram-positive bacteria at a site may include applying a polymeric superporous hydrogel as disclosed herein to the site. The references to the methods of treatment by therapy or surgery or in vivo diagnosis methods in the description are to be interpreted as references to compounds, pharmaceutical compositions and medicaments of the present invention for use in those methods.

In some embodiments, applying the polymeric superporous hydrogel as disclosed herein to the site includes applying a wound dressing incorporating the polymeric superporous hydrogel to the site.

According to a further aspect of the present disclosure yet still, a method of use of a polymeric superporous hydrogel as an antimicrobial agent is disclosed herein.

These and other features of the present disclosure will become more apparent from the following description of the illustrative embodiments.

## BRIEF DESCRIPTION OF THE DRAWINGS

The invention described herein is illustrated by way of example and not by way of limitation in the accompanying figures. For simplicity and clarity of illustration, elements illustrated in the figures are not necessarily drawn to scale. For example, the dimensions of some elements may be exaggerated relative to other elements for clarity. Further, where considered appropriate, reference labels have been repeated among the figures to indicate corresponding or analogous elements.

FIG. 1A depicts a magnified (e.g., 50× magnified) scanning electron microscope (SEM) image of at least one cross-section of freeze-dried gold sputter coated PVA-Gantrez®AN 0.5% $NaHCO_3$ hydrogel foam;

FIG. 1B depicts a magnified (e.g., 350x magnified) SEM image of at least one cross-section of freeze-dried gold sputter coated PVA-Gantrez®AN 0.5% $NaHCO_3$ hydrogel foam;

FIG. 2 depicts a graphical representation of swelling kinetics in deionized water for 0.5, 0.7, and 1% w/v $NaHCO_3$ PVA-Gantrez®AN foams and control (0%) over a 24 hour time period;

FIG. 3 depicts a graphical representation of swelling of autoclaved hydrogel foams and control in simulated wound fluid (SWF) over a 9 day time period;

FIG. 4A depicts a graphical representation of the level of adhesion to skin (force of detachment) of PVA-Gantrez® AN SPHs and conventional hydrogel as control;

FIG. 4B depicts a graphical representation of the level of adhesion to skin (work of adhesion) of PVA-Gantrez® AN SPHs and conventional hydrogel as control;

FIG. 4C depicts a graphical representation of the level of adhesion to skin (cohesiveness) of PVA-Gantrez® AN SPHs and conventional hydrogel as control;

FIG. 5A depicts an antimicrobial activity assessment by disk diffusion method of PVA-Gantrez® AN hydrogel foams (left) and control (Aquacel® Ag, ConvaTec Ltd, right) against *Staphylococcus aureus* ($10^4$ and $10^8$ CFU/mL);

FIG. 5B depicts a graphical representation of a growth inhibition area assessment by disk diffusion method of PVA-Gantrez® AN hydrogel foams and control (Aquacel® Ag, ConvaTec Ltd) against *Staphylococcus aureus* ($10^4$ and $10^8$ CFU/mL); and

FIG. 6 depicts PVA/PMVEMA hydrogels prepared with addition of 1 mg (a), 5 mg (b), 10 mg (c), 20 mg (d), and 30 mg (e) of $NaHCO_3$ in solution to provide PVA/PMVEMA solution mixtures.

## DETAILED DESCRIPTION

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to

effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

A number of features described below may be illustrated in the drawings in phantom. Depiction of certain features in phantom is intended to convey that those features may be hidden or present in one or more embodiments, while not necessarily present in other embodiments. Additionally, in the one or more embodiments in which those features may be present, illustration of the features in phantom is intended to convey that the features may have location(s) and/or position(s) different from the locations(s) and/or position(s) shown.

As used herein and in the appended claims, the singular forms "a," "and," and "the" include plural referents unless the context clearly dictates otherwise. Thus, for example, reference to "an agent" includes a plurality of such agents, and reference to "the polymer" includes reference to one or more polymers (or to a plurality of polymers). When referring to a number or a numerical range, the term "about" as used herein means that the number or numerical range referred to is an approximation within experimental variability (or within statistical experimental error), and thus the number or numerical range varies between 1% and 15% of the stated number or numerical range. The term "comprising" (and related terms such as "comprise" or "comprises" or "having" or "including") is not intended to exclude that which may be present in other certain embodiments, such as in embodiments of any composition or composition of matter, methods, processes, or the like, for example.

Provided herein are superporous hydrogels that, at least in some embodiments, may be adapted for use in, or otherwise incorporated into, topical compositions. In some embodiments, "hydrogel" may refer to a hydrophilic gel comprising a gel matrix and water. Additionally, in some embodiments, a topical composition may include at least one polyhydric alcohol, at least one viscosity increasing agent, and water. Furthermore, in some embodiments, the superporous hydrogels of the present disclosure may be embodied as, or otherwise included in, formulations that may be applied or administered in any suitable manner, which may include wound dressings to seal in the formulations deemed appropriate by the human patient or caregiver. Examples of such dressings include, but are not limited to, semipermeable films, foams, hydrocolloids, and calcium alginate swabs.

Exemplary polyhydric alcohols that may be present in a composition of the present embodiments include, but are not limited to, glycerol, propylene glycol, polyethylene glycol, polypropylene glycol, triethylene glycol, neopentyl glycols, triethanolamine, diethanolamine, ethanolamine, butylene glycol, polyethylene glycol, n-methyl diethanolamine, isopropanolamine, sorbitol, arabitol, erythritol, HSH, isomalt, lactitol maltitol, mannitol, xylitol, threitol, ribitol, galactitol, fucitol, iditol, inositol, volemitol, and any combination thereof. In some embodiments, a composition of the present disclosure may include glycerol.

Exemplary viscosity increasing agents that may be present in a composition of the present embodiments include, but are not limited to, a carboxypolymethylene; a polyacrylic polymer such as polyacrylic acid, a polyacrylate polymer, a cross-linked polyacrylate polymer, a cross-linked polyacrylic acid, and mixtures thereof; a cellulose ether such as hydroxyalkyl cellulose polymers such as hydroxypropyl methyl cellulose (HPMC), hydroxypropyl cellulose, hydroxyethyl cellulose, methyl cellulose, carboxymethyl cellulose, and mixtures thereof; a methacrylate; a polyvinylpyrrolidone; cross-linked polyvinyl pyrrolidone; polyvinylpyrrolidone-vinyl acetate copolymer; polyvinyl alcohol; polyethylene oxide; polyethylene glycol; polyvinyl alkyl ether-maleic acid copolymer; a carboxy vinyl polymer; a polysaccharide; a gum such as sodium alginate, carrageenan, xanthan gum, gum acacia, arabic gum, guar gum, pullulan, agar, chitin, chitosan, pectin, karaya gum, zein, hordein, gliadin, locust bean gum, tragacantha, and mixtures thereof; a protein such as collagen, whey protein isolate, casein, milk protein, soy protein, gelatin, and mixtures thereof; a starch such as maltodextrin, amylose, high amylose starch, corn starch, potato starch, rice starch, tapioca starch, pea starch, sweet potato starch, barley starch, wheat starch, waxy corn starch, modified starch (e.g. hydroxypropylated high amylose starch), dextrin, levan, elsinan, gluten, and mixtures thereof; bentonite; calcium stearate; ceratonia; colloidal silicon dioxide; dextrin; hypromellose; polycarbophil; kaolin; saponite; sorbitan esters; sucrose; sesame oil; tragacanth; potassium alginate; povidone; sodium starch glycolate; phospholipids; and any combination thereof.

The preparation of various SPHs are described in U.S. Patent No. 6,271,278. SPHs are also described by Chen, et al., in J. Biomed. Mater. Res. 44:53-62 (1999). SPHs may be synthesized by a gas blowing (or foaming) technique from a number of vinyl monomers, such as acrylamide (AM), 2-acrylamido-2-methylpropane sulfonic acid (AMPS), hydroxyethylmethacrylate (HEMA), or 3-sulfopropyl acrylate potassium salt (SPAK), for example. At least in some cases, that technique may involve adding a monomer, a crosslinker (N,N'-methylene-bis-acrylamide), a foam

stabilizer (Pluronic F127, Pluronic F105 or Silwet®), water, an acid (acrylic acid (AA) or hydrochloric acid (HCL) to adjust the pH to 5-6, for example), an initiator (such as ammonium persulfate (APS), for example), a catalyst (such as tetramethylethylenediamine (TEMED), for example), and a $NaHCO_3$ suspension (i.e., as a blowing agent) to a test tube, which may be shaken after the addition of each ingredient. The gas bubbles formed after the addition of the $NaHCO_3$ suspension may be homogeneously distributed in the monomer solution using a spatula, for example. To remove residual monomer, non-reacted crosslinker, and/or initiator impurities, the flexible foam may be washed thoroughly with water. After the purification process, a water-miscible alcohol such as, for example, ethanol, may be added and subsequently removed to dehydrate the hydrogel. Typically, as a final step, drying may be performed in an oven, such as in a vacuum oven at low temperatures, for example.

Some SPHs (e.g., early SPHs) were adapted for use with, or otherwise embodied as, gastric retention devices. Some early SPHs had characteristics in common with the so-called "superabsorbent" polymers (SAPs) initially used in agriculture and diaper manufacturing. Although both SPHs and SAPs are cross-linked polymeric networks capable of absorbing aqueous fluids up to 1000 times their weight, their internal structures differ from one another (e.g., the pores of SAPs are not interconnected but rather closed or semi-open and the pores of SPHs are interconnected). The internal structure differences resulted in, or are otherwise associated with, different swelling kinetics (e.g., size-dependent swelling kinetics for SAPs and size-independent swelling kinetics for SPHs).

Later SPHs attempted to overcome the mechanical weakness of conventional SPHs and were called "SPH composites" because of their structure. *See* Chen J. et al., Synthesis and Characterization of Superporous Hydrogel Composites, I Control. Release 2000, 65 (1-2), 73-82. The SPH composites were composed of a matrix containing dispersed particles of a "composite agent" (i.e., a cross-linked hydrophilic polymer that can absorb the reaction liquid mixture) with all the compounds needed for SPHs synthesis (such as a monomer, a crosslinker, and an initiator). SPH composites were used for intestinal and peroral drug delivery even though their response to mechanical stress was not optimal. *See* Omidian H. et al., Advances in Superporous Hydrogels, I Control. Release 2005, 102 (1), 3-12).

Even later SPHs known as "SPH hybrids" contained a water-soluble or dispersible polymer capable of undergoing physical or chemical cross-linking after the SPH network is formed to provide a fully interpenetrating network (IPN) hydrogel structure. *See* Dragan E. S., Design and Applications of Interpenetrating Polymer Network Hydrogels. A Review. Chem. Eng. 1 2014, 243, 572-590). SPH hybrids exhibited excellent elasticity and strength even in the swollen state and were widely investigated for challenging purposes such as proteins/peptides delivery.

A new technique for the production of SPHs is envisioned by the present disclosure. The methods and compositions described herein provide a cost-effective, quick, and eco-friendly procedure that involves the use of macromolecules, such as polyvinyl alcohol (PVA) and a copolymer of methyl vinyl ether and maleic anhydride, for example, and water as a solvent. In some embodiments, the copolymer of methyl vinyl ether and maleic anhydride may be embodied as, or otherwise include, Gantrez®AN.

The methods of the present disclosure may be used to produce a superporous material that provides antimicrobial activity in use, and as such, the methods and compositions herein may utilize features, steps, and/or aspects to provide benefits and/or advantages that are not attained by other methods or compositions. A simple, fast, and environmentally-friendly method for superporous hydrogel production is presented herein. The superporous hydrogel foams successfully produced according to this method offer promising materials for wound management as wound dressings, at least in some embodiments.

## Polymers

In one aspect, a superporous hydrogel of the present disclosure is prepared from one or more polymers having one or more functional groups which are capable of co-reacting in a condensation reaction. In some embodiments, the superporous hydrogel is prepared from a single multifunctional polymer including two or more functional groups which are capable of reacting in a condensation reaction. Additionally, in some embodiments, the superporous hydrogel is prepared from two or more polymers, and each polymer includes one or more functional groups capable of reacting in a condensation reaction. The functional groups include, but are not limited to, alcohols, carboxylic acids, carbonyls, esters, amides, anhydrides, and combinations thereof.

In some embodiments, the superporous hydrogel is prepared from two or more polymers, each polymer includes one or more functional groups capable of reacting in a condensation reaction wherein the polymers are selected from polyvinyl alcohol (PVA)

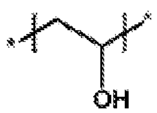

; polyacrylic acid (PAA)

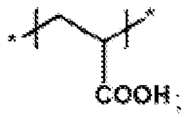

copolymer of poly(alkylvinylether/maleic acid) such as poly(methyl ether-*alt*-maleic anhydride) (PMVEMA)

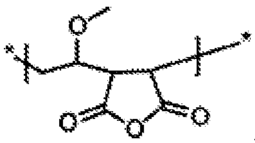

and poly(methyl vinyl ether-*alt*-maleic acid) (PMVEMAC)

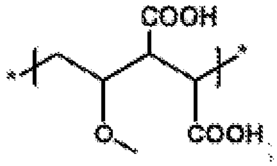

copolymer of poly(styrene/maleic anhydride) such as poty(styrene-co-maleic anhydride) (SMA)

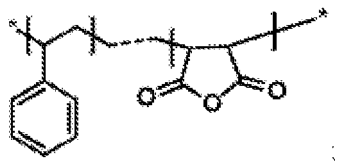

2-hydroxyethyl cellulose (HEC)

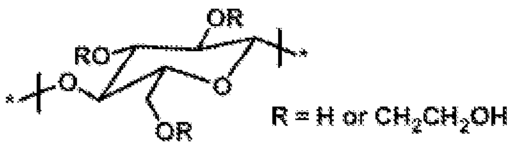

; and combination thereof.

In some embodiments, the superporous hydrogel is prepared from PVA and PMVEMAC. In some embodiments still, the superporous hydrogel is prepared from PVA and SMA. In some embodiments yet still, the superporous hydrogel is prepared from HEC and PMVEMA. Further, in some embodiments, the superporous hydrogel is prepared from HEC and PMVEMAC. Finally, in some embodiments, the superporous hydrogel is prepared from HEC and SMA.

In some embodiments, the molar ratio of a first polymer to a second polymer is at least about 1: 1. In some embodiments, the molar ratio may be in a range of about 1:1 to about 2:1, such as, for example, about 1.1:1, about 1.2:1, about 1.3:1, about 1.4:1, about 1.5:1, about 1.6:1, about 1.7:1, about 1.8:1, about 1.9:1, or about 2:1.

## Blowing Agents

In one aspect, a superporous hydrogel of the present disclosure is prepared from one or more polymers having one or more functional groups which are capable of co-reacting in a condensation reaction and gas bubbles. In some embodiments, the gas bubbles are formed by a mechanical gas blowing method. Additionally, in some embodiments, the gas bubbles are formed by a chemical gas blowing method by addition of a blowing agent. The gas bubbles include air, at least in some embodiments. Further, in some embodiments, the gas bubbles include carbon dioxide.

In some embodiments, a carbonate is used to generate bubbles. The carbonate may be added to the copolymers

as a solid and not as an aqueous solution, at least in some embodiments. Additionally, in some embodiments, when using a carbonate aqueous solution, the carbon dioxide bubbles generated may be very large and may escape the reaction mixture too quickly before gelation. Further, in some embodiments, the carbonate is sodium carbonate, sodium bicarbonate, potassium carbonate, potassium bicarbonate, calcium carbonate, calcium bicarbonate, or lithium carbonate. In some embodiments, the carbonate is sodium bicarbonate.

In some embodiments, the amount of blowing agent is between about 0.1% w/v and about 1% w/v. In such embodiments, the amount of blowing agent may be about 0.1% w/v, about 0.2% w/v, about 0.3% w/v, about 0.4% w/v, about 0.5% w/v, about 0.6% w/v, about 0.7% w/v, about 0.8% w/v, about 0.9% w/v, or about 1% w/v.

## Solvent

In one aspect, a superporous hydrogel of the present disclosure is prepared from one or more polymers having one or more functional groups which are capable of co-reacting in a condensation reaction, gas bubbles, and water. In some embodiments, no organic solvents are used to prepare the superporous hydrogel.

## Foam Stabilizer

In some embodiments, the superporous hydrogel includes a foam stabilizer. In such embodiments, the foam stabilizer may be selected from albumin, gelatin, Pluronics® [(polyethylene oxide)-poly(propylene oxide)-poly(ethylene oxide) (PEO-PPO-PEO) tri-block copolymers), Silwet® (polyalkyleneoxidemodified poly(dimethyl siloxane) surfactants), sodium dodecyl sulfate, Span®, Triton®, and Tween®. Additionally, in such embodiments, the foam stabilizer may be Pluronic F127 (PF127). In other embodiments, no foam stabilizer is used.

## Properties

### Pore size

In some embodiments, the amount of blowing agent controls the pore size and the porosity of the superporous hydrogel. Additionally, in some embodiments, the pore size is nonhomogeneous and in one of the following ranges: about 5 μm to about l mm, about 5 μm to about 100 μm, about 10 μm to about 60 μm, about 10 μm to about 200 μm, about 200 μm to about 500 μm, about 400 μm to about 700 μm, or about 600 μm to about 900 μm. Furthermore, in some embodiments, the pore size is about 5 μm, about 10 μm, about 20 μm, about 30 μm, about 40 μm, about 50 μm, about 60 μm, about 70 μm, about 80 μm, about 90 μm, about 100 μm, about 110 μm, about 120 μm, about 130 μm, about 140 μm, about 150 μm, about 160 μm, about 170 μm, about 180 μm, about 190 μm, about 200 μm, about 250 μm, about 300 μm, about 350 μm, about 400 μm, about 450 μm, about 500 μm, about 550 μm, about 600 pm, about 650 μm, about 700 μm, about 800 μm, about 850 μm, about 900 μm, about 950 μm, or about 1 mm.

### Density

In some embodiments, the density of the superporous hydrogel is in the range of about 0.05 to about 5 g/cm$^3$, or about 0.05 to about 4 g/cm$^3$, or about 0.05 to about 3 g/cm$^3$, or about 0.05 to about 2 g/cm$^3$, or about 0.05 to about 1.5 g/cm$^3$, or about 0.05 to about 1 g/cm$^3$.

### Absorption capacity

In some embodiments, the absorption capacity of the superporous hydrogel is in the range of about 30% and about 20000%, or about 100% and about 10000%, or about 300% and about 10000%.

### Swelling properties

In some embodiments, the superporous hydrogels described herein exhibit very high and fast swelling properties. In some embodiments, the swelling ratio after 24 hours is greater than about 3000%, greater than about 3500%, greater than about 4000%, greater than about 4500%, greater than about 5000%, greater than about 5500%, greater than about 6000%, greater than about 6500%, greater than about 7000%, greater than about 7500%, greater than about 8000%, or greater than about 8500%. Additionally, in some embodiments, the swelling ratio after 24 hours is between about 4000% and about 8500%. Further, in some embodiments, the swelling ratio after 24 hours is about 4000%. In some embodiments still, the swelling ratio after 24 hours is about 7000%. Finally, in some embodiments, the swelling ratio after 24 hours is about 8500%.

Mechanical properties

In some embodiments, the mechanical properties of the superporous hydrogels of the present disclosure may be advantageous in one or more respects.

*Elastic modulus (Young's modulus):*

The elastic modulus (also known as the tensile modulus or Young's modulus) is a number that measures the resistance of an object or substance to being deformed elastically (i.e., non-permanently) when a force is applied to it. The elastic modulus of an object is defined as the slope of its stress-strain curve in the elastic deformation region. It should be appreciated that a stiffer material will have a higher elastic modulus than a material having less stiffness.

In some embodiments, a polymeric superporous hydrogel exhibits an elastic modulus (Young's modulus) between about $1\times10^{-5}$ N/mm$^2$ and about $4\times10^{-5}$ N/mm$^2$. Additionally, in some embodiments, the polymeric superporous hydrogel exhibits an elastic modulus (Young's modulus) of at least about $1\times10^{-5}$ N/mm$^2$, at least about $1.5\times10^{-5}$ N/mm$^2$, at least about $2\times10^{-5}$ N/mm$^2$, at least about $2.5\times10^{-5}$ N/mm$^2$, at least about $3\times10^{-5}$ N/mm$^2$, at least about $3.5\times10^{-5}$ N/mm$^2$, or at least about $4\times10^{-5}$ N/mm$^2$. In some embodiments still, the polymeric superporous hydrogel exhibits an elastic modulus (Young's modulus) between about $1\times10^{-5}$ N/mm$^2$ and about $3\times10^{-5}$ N/mm$^2$. In some embodiments yet still, the polymeric superporous hydrogel exhibits an elastic modulus (Young's modulus) between about $2.9\times10^{-5}$ N/mm$^2$ and about $3.7\times10^{-5}$ N/mm$^2$. In some embodiments, the polymeric superporous hydrogel exhibits an elastic modulus (Young's modulus) between about $1.4\times10^{-5}$ N/mm$^2$ and about $3.8\times10^{-5}$ N/mm$^2$.

*Elongation at break:*

Elongation at break, also known as fracture strain, is the ratio between changed length and initial length after breakage of the test specimen. The elongation at break expresses the capability of a material to resist changes of shape without crack formation. The elongation at break is determined by tensile testing.

In some embodiments, a polymeric superporous hydrogel exhibits between about 44% and about 100% elongation at break. Additionally, in some embodiments, the polymeric superporous hydrogel exhibits at least about 44%, at least about 45%, at least about 50%, at least about 55%, at least about 60%, at least about 65%, at least about 70%, at least about 75%, at least about 80%, at least about 85%, at least about 90%, at least about 95%, or at least about 100% elongation at break. In some embodiments still, the polymeric superporous hydrogel exhibits between about 44% and about 72% elongation at break. In some embodiments yet still, the polymeric superporous hydrogel exhibits between about 80% and about 96% elongation at break. Finally, in some embodiments, the polymeric superporous hydrogel exhibits between about 69% and about 96% elongation at break.

*Tensile stress at break:*

The tensile stress at break is the tensile stress at the moment at which a test specimen tears, breaks, or forms a crack. Like elongation at break, the tensile stress at break is determined by tensile testing.

In some embodiments, a polymeric superporous hydrogel exhibits a tensile stress at break between about $0.3\times10^{-5}$ N/mm$^2$ and about $1.3\times10^{-4}$ N/mm$^2$. Additionally, in some embodiments, the polymeric superporous hydrogel exhibits a tensile stress at break of at least about $0.3\times10^{-5}$ N/mm$^2$, at least about $0.4\times10^{-5}$ N/mm$^2$, at least about $0.5\times10^{-5}$ N/mm$^2$, at least about $0.6\times10^{-5}$ N/mm$^2$, at least about $0.7\times10^{-5}$ N/mm$^2$, at least about $0.8\times10^{-5}$ N/mm$^2$, at least about $0.9\times10^{-5}$ N/mm$^2$, at least about $1\times10^{-5}$ N/mm$^2$, at least about $2\times10^{-5}$ N/mm$^2$, at least about $3\times10^{-5}$ N/mm$^2$, at least about $4\times10^{-5}$ N/mm$^2$, at least about $5\times10^{-5}$ N/mm$^2$, at least about $6\times10^{-5}$ N/mm$^2$, at least about $7\times10^{-5}$ N/mm$^2$, at least about $8\times10^{-5}$ N/mm$^2$, at least about $9\times10^{-5}$ N/mm$^2$, at least about $1\times10^{-4}$ N/mm$^2$, at least about $1.1\times10^{-4}$ N/mm$^2$, at least about $1.2\times10^{-4}$ N/mm$^2$, or at least about $1.3\times10^{-4}$ N/mm$^2$. In some embodiments still, the polymeric superporous hydrogel exhibits a tensile stress at break between about $2.1\times10^{-5}$ N/mm$^2$ and about $7.3\times10^{-5}$ N/mm$^2$. In some embodiments yet still, the polymeric superporous hydrogel exhibits a tensile stress at break between about $0.7\times10^{-4}$ N/mm$^2$ and about $1.3\times10^{-4}$ N/mm$^2$. Finally, in some embodiments, the polymeric superporous hydrogel exhibits a tensile stress at break between about $0.3\times10^{-5}$ N/mm$^2$ and about $1.9\times10^{-5}$ N/mm$^2$.

*Maximum strength:*

In some embodiments, a polymeric superporous hydrogel exhibits a maximum strength between about $3\times10^{-2}$ N/mm and about $6.3\times10^{-2}$ N/mm. Additionally, in some embodiments, the polymeric superporous hydrogel exhibits a maximum strength between about $3\times10^{-2}$ N/mm and about $4.4\times10^{-2}$ N/mm. In some embodiments still, the polymeric superporous hydrogel exhibits a maximum strength between about $3.8\times10^{-2}$ N/mm and about $5.2\times10^{-2}$ N/mm. In some

embodiments yet still, the polymeric superporous hydrogel exhibits a maximum strength between about $3.1\times10^{-2}$ N/mm and about $6.3\times10^{-2}$ N/mm.

Antimicrobial activity

In some embodiments, a polymeric superporous hydrogel exhibits intrinsic antimicrobial activity. Additionally, in some embodiments, the polymeric superporous hydrogel exhibits intrinsic antimicrobial activity against Gram-positive bacteria such as *Staphylococcus aureus,* for example. In some embodiments still, the antimicrobial activity of the superporous hydrogel is comparable to the antimicrobial activity of dressings containing antimicrobial agents, such as silver ions and iodine, for example.

**Method of preparing a superporous hydrogel**

A process for the preparation of a superporous hydrogel according to the present disclosure includes crosslinking one or more polymers having one or more functional groups which are capable of co-reacting in a condensation reaction in the presence of a blowing agent. The crosslinking is achieved without the use of any extra crosslinking agent. The crosslinking is achieved by mixing the one or more polymers at elevated temperature and elevated pressure. The process may be carried out in an autoclave. The process may alternatively be carried out in a pressure cooker. The cross linking may alternatively be carried out by irradiating the solution with microwave radiation.

In some embodiments, the process for the preparation of the superporous hydrogel is conducted at elevated temperatures. Additionally, in some embodiments, the process for the preparation of the superporous hydrogel is carried out at a temperature sufficient for the polymers to crosslink without being so high that substantial degradation of the polymers takes place. In some embodiments still, the process for the preparation of the superporous hydrogel is conducted at between about 50 °C and about 200 °C, or between about 100 °C and about 150 °C. In some embodiments yet still, the process for the preparation of the superporous hydrogel is conducted at about 80 °C, about 85 °C, about 90 °C, about 95 °C, about 100 °C, about 105 °C, about 110 °C, about 115 °C, about 120 °C, about 125 °C, about 130 °C, about 135 °C, about 140 °C, about 145 °C, or about 150 °C.

In some embodiments, the process for the preparation of the superporous hydrogel is conducted at elevated pressures. Additionally, in some embodiments, the process for the preparation of the superporous hydrogel is conducted at a pressure between about atmospheric pressure and about 220 psi, such as at about 14 psi, about 15 psi, about 16 psi, about 17 psi, about 18 psi, about 19 psi, about 20 psi, about 30 psi, about 40 psi, about 50 psi, about 60 psi, about 70 psi, about 80 psi, about 90 psi, about 100 psi, about 110 psi, about 120 psi, about 130 psi, about 140 psi, about 150 psi, about 160 psi, about 170 psi, about 180 psi, about 190 psi, about 200 psi, about 210 psi, or about 220 psi. 1 psi converts to 6894.8 Pa. In some embodiments still, the process for the preparation of the superporous hydrogel is conducted at a pressure between about atmospheric pressure and about 100 psi. In some embodiments yet still, the process for the preparation of the superporous hydrogel is conducted at a pressure between about atmospheric pressure and about 40 psi. Finally, in some embodiments, the process for the preparation of the superporous hydrogel is conducted at a pressure between about 15 psi and about 40 psi.

In some embodiments, the process for the preparation of the superporous hydrogel is carried out for a sufficient period of time for the polymers to crosslink without being so long that substantial degradation of the polymers takes place. Additionally, in some embodiments, the reaction is carried out from about 10 min to about 120 min, or from about 30 min to about 60 min, such as about 30 min, about 35 min, about 40 min, about 45 min, about 50 min, about 55 min, or about 60 min.

**Applications**

The superporous hydrogel compositions described herein may suitably be used in a range of skin contact or covering applications, such as applications in which compositions are brought into contact with skin or with an intermediate interface between the compositions and the skin, for example. As described herein, skin contact or covering applications may include, or otherwise be embodied as, a wide range of applications associated with substantially whole/intact dermal tissue and/or compromised, broken, or damaged dermal tissue. As such, the superporous hydrogel compositions of the present disclosure may have a broad range of dermatological uses. For example, in some embodiments, the superporous hydrogel compositions described herein may be used to treat wet eczema, weeping and denuded tissue (e.g., wet leg syndrome or other conditions associated with chronic venous insufficiency), incontinence associated dermatitis (IAD), urinary or fecal incontinence, or conditions associated with fecal, urinary, or venous catheter usage. Additionally, in some embodiments, the superporous hydrogel compositions described herein may be adapted to treat complications and/or conditions associated with menstruation.

The superporous hydrogel compositions described herein may each be unsupported or supported on a part of

a larger article (e.g., a backing structure) for a particular use. The compositions may suitably be in the form of sheets, coatings, membranes, composites, laminates, or the like. Applications contemplated by the present disclosure include, but are not limited to, patches, tapes, bandages, dressings, absorbent pads for absorbing body fluids (e.g., lactation pads for nursing mothers), adhesive flanges and tabs for fecal collection receptacles, ostomy devices, and other incontinence devices.

For the purposes of the present disclosure, a wound or wound site to which the superporous hydrogel may be applied undergoes a multi-phase healing process that, at least in some embodiments, includes four phases: hemostasis, inflammation, proliferation, and maturation. Managing exudate during the hemostasis and proliferation phases of wound healing is vital to prevent maceration, recurring infection, and other adverse effects on the patients. Wound dressings that exert negative pressure at the site of wound absorb excess excaudate and promote healing. Exerting and maintaining negative pressure of wound dressings is ideal for treating and managing various types of chronic or acute dermal wounds stemmed from infection, ulcers, burns, abrasions, incisions, lacerations, punctures, avulsions, and amputations.

In some embodiments, the superporous hydrogel is included in a wound dressing. In such embodiments, the wound dressing has a plurality of layers and one of the layers includes the superporous hydrogel. Additionally, in some embodiments, the wound dressing also includes an active agent. The active agent may be included in the same layer as the superporous hydrogel or may be included in a different layer. In some embodiments, the active agent is selected from anti-inflammatory agents, anesthetics, and combination thereof. For example, in some embodiments, the active agent is a topical corticosteroid selected from hydrocortisone, clobetasone butyrate, and betamethasone dipropionate. In some embodiments still, the active agent is lidocaine or benzocaine. In some embodiments yet still, the active agent is an antimicrobial agent that may be selected from metal ions (such as silver) and halide ions (such as chloride or iodide), though the antimicrobial agent need not require pairing of a metal ion with a halide ion. The active agent may also be selected from polyhexamethylene biguanide (PHMB), chlorhexidine, and octalenidine, at least in some embodiments.

Additionally, in some embodiments, when incorporated into a wound dressing or the like having a plurality of layers, for example, the superporous hydrogel may be capable of movement within the layer(s) in which the hydrogel is incorporated. In such embodiments, the plurality of layers or sheets may have a number of orientations relative to one another to facilitate such movement and/or to improve wound exudate handling and management. In one example, one or more layers or sheets of a wound dressing may be oriented substantially perpendicular to a wound site such that movement of the hydrogel within the layer(s) in which the hydrogel is incorporated facilitates wound exudate wicking and/or moisture evaporation. In some cases, the dressing or the like incorporating the superporous hydrogel may be embodied as, or otherwise include, a high Moisture Vapor Transmission Rate (MVTR) dressing. Additionally, in some cases, the dressing or the like incorporating the superporous hydrogel may include, or otherwise be embodied as, a diagnostic device such as a sensor or the like. Furthermore, in some cases, the dressing or the like incorporating the superporous hydrogel may be embodied as, or otherwise included in, a device adapted for negative pressure wound therapy (NPWT) applications, such as Avelle-type applications, for example.

It should be appreciated that the superporous hydrogel of the present disclosure is not limited to applications in which the hydrogel is incorporated into one or more layer(s) of a dressing or the like. For instance, in some embodiments, the superporous hydrogel may be embodied as, or otherwise included in, a three-dimensional foam having a form that may be substantially maintained when incorporated into a device. Of course, in other embodiments, the superporous hydrogel may be provided in another suitable form.

**EXAMPLES** (not according to the invention)

**Materials**

Medium molecular weight (51.3 kDa) PVA of 98-99% hydrolysis was purchased from Alfa Aesar (UK). PVA actual degree of deacetylation was found to be 98.7% by $^1$H NMR. Nutrient agar used for the antimicrobial test was purchased from Oxoid Ltd UK. Gantrez® AN (Average Mw about 216 kDa) and all the other chemicals used were purchased from Sigma-Aldrich (UK). Aquacel® Ag dressing was kindly provided by ConvaTec Ltd. to be used as control during the antimicrobial studies.

Example 1: **Hydrogel foam synthesis**

Medium molecular weight (51.3 kDa) PVA of 98-99% hydrolysis was dissolved in deionized water at about 90 °C for approximately 6 hours. PMVEMA (Gantrez® AN, Average Mw about 216 kDa) was dissolved in deionized water at about 50 °C overnight.

The aqueous solutions of PVA (16.6% w/v) and PMVEMA (14.8% w/v) were mixed together in 15 mL glass vials using a rotator mixer for 3 hours. Formulations with a final molar ratio of 2:1 (base moles of PVA per base moles

of Gantrez® AN) were prepared. Sodium bicarbonate ($NaHCO_3$) powder was homogeneously spread using a spatula on the surface of glass petri dishes (8 cm diameter). The mixtures were then poured into the petri dishes. Foams with 0.5, 0.7, and 1% w/v $NaHCO_3$ were produced. The petri dishes were then quickly sealed and autoclaved for 30 minutes at 121°C (CertoClav Multicontrol 12 L).

**Comparative Example 1: hydrogel formation using $NaHCO_3$ in solution**

20 mL mixtures containing PMVEMA and PVA were prepared by mixing individual polymer solutions in 1:1 molar ratio and placed on a tumbling mixer for 3.5 hours to ensure their thorough mixing. Then, 1 mg, 5 mg, 10 mg, 20 mg, and 30 mg amounts of $NaHCO_3$ were added and the mixtures subsequently mixed in a tumble mixer for 1 hour until complete salt dissolution. The glass vials were then sealed and autoclaved at 125 °C for 1.5 hours. Formation of gas-filled large cavities within these hydrogels was observed but these cavities were irregularly distributed within the samples. The hydrogel samples are shown in FIG. 6. The vial labeled (a) corresponds to the hydrogel sample including 1 mg of $NaHCO_3$. The vial labeled (b) corresponds to the hydrogel sample including 5 mg of $NaHCO_3$. The vial labeled (c) corresponds to the hydrogel sample including 10 mg of $NaHCO_3$. The vial labeled (d) corresponds to the hydrogel sample including 20 mg of $NaHCO_3$. The vial labeled (e) corresponds to the hydrogel sample including 30 mg of $NaHCO_3$.

**Example 2: Scanning electron microscopy (SEM) analysis**

A FEI Quanta FEG 600 Environmental Scanning Electron Microscope (ESEM) was used to analyze the porous internal structure of PVA-Gantrez® AN hydrogel foams of Example 1. Cross-sections of gold sputter coated freeze-dried samples (which are shown in FIG. 1A and 1B) were used. Liquid nitrogen ($N_2$) was used to freeze the samples in order to keep their structure unaltered.

Due at least in part to the acidic pH of the reaction mixture, $CO_2$ bubbles rapidly generated by $NaHCO_3$ move from the bottom of the petri dish towards the surface of the polymeric mixture. However, the high viscosity of the solution slows down the gas bubbles such that escape of the bubbles from the hydrogel matrix is substantially prevented. Instead, the gas bubbles are substantially retained in the crosslinked hydrogel matrix that forms at the same time during the autoclaving step. A sterile, homogenous, superporous, sponge-like hydrogel is then formed. An interconnected structure of open pores may be observed based on the SEM images of the cross-sections of freeze-dried foam samples shown in FIGS. 1A and 1B. As indicated above, FIG. 1A corresponds to a SEM image of a cross-section of a freeze-dried gold sputter coated PVA-Gantrez®AN 0.5%$NaHCO_3$ hydrogel foam that is magnified 50x, whereas FIG. 1B corresponds to a SEM image of a cross-section of a freeze-dried gold sputter coated PVA-Gantrez®AN 0.5%$NaHCO_3$ hydrogel foam that is magnified 350x.

**Example 3: Swelling studies**

The swelling behavior of PVA-Gantrez® AN superporous hydrogels of Example 1 was assessed in deionized water and in simulated wound fluid (SWF) containing 8.298 g/l (water) of sodium chloride (NaCl) and 0.368 g/l (water) of calcium chloride (CaCh). The initial weight of each sample *(Wi)* was accurately recorded. The samples were kept in excess water for 24 hours to study their fast swelling in that medium, and the duration of the experiment was extended to a longer period (9 days) when conducted in SWF. In both cases, the samples were weighed regularly and excess unbound medium was wiped away using filter paper. The swelling ratio (SR in %) of the PVA-Gantrez® AN foams was calculated using the following formula:

$$SR = [(W_S - W_i) / W_i] \times 100 \qquad (1)$$

Where *Ws* is the weight of the swollen sample.

A 24 hour swelling test in deionized water was performed to assess the profile of the initial swelling of autoclaved superporous PVA-Gantrez®AN hydrogels. FIG. 2 illustrates swelling kinetics in deionized water for 0.5% w/v, 0.7% w/v, and 1% w/v $NaHCO_3$ PVA-Gantrez®AN foams and control (0%) during the 24 hour period. An asterisk (*) indicates significant statistical difference (($p<0.05$) among samples) at a particular time point. The materials exhibit very high and fast swelling in deionized water. That swelling increases accordingly with the amount of $NaHCO_3$ contained in the formulation, and consequently, also with the $CO_2$ spontaneously generated in the reaction mixture. The test using simulated wound fluid (SWF) was prolonged for 9 days to determine a period over which a dressing made of the materials produced could remain stable on a hypothetical wound exudating at a very high rate. FIG. 3 illustrates swelling of autoclaved hydrogel foams and control in simulated wound fluid (SWF) over the 9 day period.

The most commonly used wound dressings, such as plain gauze, for example, need to be removed and changed

daily with associated pain and trauma for the fragile, newly formed skin often adhering to the applied products. Dressings incorporating hydrogels typically allow for less frequent removal (up to 3 days) and substantially constant wound bed monitoring due to their transparency. Therefore, hydrogel materials with enhanced swelling abilities like SPHs offer further advantages with regard to removal frequency and wound bed monitoring. As shown in FIG. 3, the swelling ratio of the autoclaved foams, even if still quite high, is significantly reduced in simulated wound fluid (SWF) due in part to the difference in the osmotic pressure between the gel matrix and the external medium containing high amounts of mobile ions. In this case, the different $NaHCO_3$ content does not affect the swelling ratio of the three formulations (i.e., no significant statistical difference was found at any time point).

**Example 4: Tensile test**

The mechanical properties of the autoclaved hydrogel foams of Example 1 were studied using a Texture Analyzer XT Plus (T.A., Stable Micro Systems Ltd, UK). 2 cm length rectangular samples (each having 4 mm width) were cut from initial 8 cm diameter foams. An extension of 100 mm/min to the break was applied to all samples with an initial "grip-to-grip" separation of 30 mm. Strength-strain curves were plotted during the test by T.A. software (T.A. Exponent).

Several variables can affect the mechanical properties of SPHs prepared according to conventional methods, such as the monomer or crosslinker used, the amount of foaming agent added, or any post-synthesis step, for example. However, the SPHs presented herein were produced by autoclaving without any monomers and/or crosslinkers, and without undergoing any treatment after gelation. The tensile performance of PVA-Gantrez® AN foam samples containing different amounts of $NaHCO_3$ and a conventional hydrogel as control (produced using the same technique but without the addition of NaHCO3) using a T.A. test in the "film stretching" set-up are compared below in Table 1. Data are shown as mean ± standard deviation. Statistical significance ($p<0.05$) was calculated for tensile stress at break, maximum strength and elongation at break and Young's modulus values obtained for all hydrogel foams (a-c) and control (d). Letters after each value indicate which other composition the sample is significantly different from.

**Table 1 Mechanical properties of PVA-Gantrez® AN SPHs**

| Concentration of $NaHCO_3$ | Tensile stress at break ($N/mm^2$) | Maximum strength (N/mm) | Elongation at break (%) | Young's Modulus ($N/mm^2$) |
|---|---|---|---|---|
| [a]0% | $(2.2 \pm 1.6) \times 10^{-4}$ | $(6.3 \pm 0.9) \times 10^{-2}$ | 48.4 ± 10.2 | $(1.0 \pm 0) \times 10^{-4}$ |
| [b] 0.5% | $(4.7 \pm 2.6) \times 10^{-5}$ | $(3.7 \pm 0.7) \times 10^{-2}$ | 58.7 ± 14.1 | $(2.0 \pm 1.0) \times 10^{-1}$ (a) |
| [c] 0.7% | $(1.0 \pm 0.3) \times 10^{-4}$ | $(4.5 \pm 0.7) \times 10^{-2}$ | 89.1 ± 7.4 (a) | $(3.3 \pm 0.4) \times 10^{-5}$ (a) |
| [d] 1.0% | $(1.1 \pm 0.8) \times 10^{-5}$ | $(4.7 \pm 1.6) \times 10^{-2}$ | 82.8 ± 13.8 (a) | $(2.6 \pm 1.2) \times 10^{-5}$ (a) |

As shown in Table 1, the resulting materials are less stiff and more flexible when the foaming agent is added and the superporous foam internal structure is then formed. The elongation at break, which is the deformation or strain that the material undergoes at fracture as indicated above, is significantly improved in the SPHs compared with the control. The foam samples exhibit more desirable values of Young's modulus as well. However, the difference in the amount of $NaHCO_3$ contained in the foams did not correspond to a statistically significant difference in the Young's modulus results of the foams. No statistically significant difference was found for tensile stress at break and maximum strength data collected during the test.

**Example 5: PVA-Gantrez® AN superporous hydrogels adhesion to skin**

A Texture Analyzer (T.A.) was used to perform the adhesion test described in the following reference: Calò E. et al., Antimicrobial Hydrogels Based on Autoclaved Poly(vinyl Alcohol) and Poly(methyl Vinyl Ether-Alt- Maleic Anhydride) Mixtures for Wound Care Applications, RSC Adv. 2016, 6, 55211-55219. 20 mm diameter discs of superporous hydrogels of Example 1 were attached to the probe of the instrument and 5 cm × 5 cm porcine skin sections (from the shaved back of the animal, provided by P.& D. Jennings Butchers, Hurst, UK) were placed on a metallic platform. The settings used for the analysis were the following: pre-speed test 1.0 mm/s; test-speed 0.5 mm/s; post-test speed 1.0 mm/s; applied force 0.5 N; return distance 10.0 mm; contact time 60.0 s; trigger type auto; trigger force 0.1 N; and return distance of 10.0 mm. Adhesive strength, total work of adhesion, and cohesiveness data for the hydrogel foams produced by autoclaving were collected by the T.A. software (T.A. Exponent).

The level of adhesion to skin represents an important characteristic to be considered when designing a wound

management product. An adhesion test by a T.A. test and shaved porcine skin as the skin model was performed to evaluate the adhesiveness of the hydrogel foams produced by autoclaving. FIG. 4A illustrates the force required to detach the hydrogel foam samples (i.e., 0.5% w/v, 0.7% w/v, and 1% w/v $NaHCO_3$ PVA-Gantrez®AN foams and control (0%)) from the skin. FIG. 4B illustrates the total work of adhesion of the samples, which is defined as the area under the force versus distance curve. FIG. 4C illustrates the cohesiveness of the samples, which represents the distance traveled by the samples until complete detachment. An asterisk (*) indicates a significant statistical difference ($p<0.05$)) at a particular time point.

FIG. 4B illustrates that the level of adhesion significantly decreases for the PVA-Gantrez® AN foam samples compared to the control. This may be attributed to the irregular surface presented by the foams, as well as the higher degree of physical and chemical crosslinking achieved in these materials due to a limited availability of groups capable of hydrogen bonding with functional groups present on the skin (according to the adsorption theory of adhesion). No significant statistical difference was found among the foam compositions.

**Example 6: Antimicrobial activity of hydrogel foams tablets**

PVA-Gantrez® AN superporous hydrogels of example 1 were freeze-dried and compressed in order to obtain disc-shaped tablets (10 mm diameter and 3 mm width) to perform a disc diffusion test in view of the techniques disclosed in the following references: Hallander H. 0., et al., Identification of Cephalosporin-Resistant Staphylococcus Aureus with the Disc Diffusion Method, Antimicrob. Agents Chemother, 1972, 1 (5), 422-426; Boonkaew B., et al., Antimicrobial Efficacy of a Novel Silver Hydrogel Dressing Compared to Two Common Silver Burn Wound Dressings: Acticoat' and PolyMem Silver(®), Burns 2014, 40 (1), 89-96. *Staphylococcus aureus* NCTC 8532 (National Collection of Type Cultures) was used to test the antimicrobial activity of the SPHs of Example 1. *Staphylococcus aureus* was grown overnight at 37 °C (shaking at 200 rpm) in nutrient broth. Sterilized nutrient agar (prepared as indicated by the manufacturer) was used to prepare the plates, which were inoculated with two different concentrations of bacteria: $10^8$ CFU/mL and $10^4$ CFU/mL. The samples were placed exactly in the center of the agar plates and incubated for 24 hours at 37°C. The clear zone surrounding the tablets, related to bacteria growth inhibition, was measured using a ruler and recorded.

Bacterial colonization is a recurring reason for delays in the healing process. In some cases, such colonization may intensify into a "critical colonization" condition associated with severe pain, which may be a precursor to infection. The use of wound dressings containing antimicrobial agents, such as silver ions and iodine, for example, is often preferred over the administration of systemic antibiotics, especially when the patient is affected by circulatory pathologies that may prevent the drug from having the required therapeutic effect and has chronic, infected wounds. However, that use is typically dependent on the type of compromised wound presented and any possible allergies of the patient.

The antimicrobial activity of autoclaved PVA-Gantrez® AN foams was assessed using a disk diffusion test against *Staphylococcus aureus,* which is one of the opportunistic Gram-positive bacteria most commonly present in chronic wounds. That test allows a rapid evaluation of bacterial growth inhibition. In this case, the test was conducted using freeze-dried samples compressed in the form of disc-shaped tablets to better visualize the results. FIG. 5A illustrates the antimicrobial activity assessment by disk diffusion method of PVA-Gantrez® AN hydrogel foams (0.7% $NaHCO_3$) (left) and control (Aquacel® Ag, ConvaTec Ltd, right) against *Staphylococcus aureus* ($10^4$ and $10^8$ CFU/mL). FIG. 5B illustrates graphically the growth inhibition area assessment corresponding to the disk diffusion method associated with FIG. 5A Growth inhibition area was measured with a scale bar of 2 cm. An asterisk (*) indicates a significant statistical difference ($p<0.05$)) at a particular time point.

A clear zone with no bacteria proliferation is observed around the tablets. The bacteria growth inhibition due to the presence of the SPHs dressings was higher when the bacterial concentration was lower ($10^4$ CFU/mL) and comparable with the control containing silver. The antimicrobial activity of the samples is related to the presence of Gantrez® AN and to its low pH (4-5), which creates a hostile growth environment for *Staphylococcus aureus.* Therefore, the antimicrobial effect decreased accordingly with the increase in the pH, and samples containing 1% w/v $NaHCO_3$ (having a pH of about 5) did not show this effect at the bacterial concentration of $10^8$ CFU/mL.

## Claims

**1.** A wound dressing comprising a polymeric superporous hydrogel comprising:

a copolymer of poly(alkylvinylether/maleic acid), or a copolymer of poly(styrene/maleic anhydride);
polyvinyl alcohol (PVA) or hydroxyethylcellulose (HEC); and
a plurality of pores.

**2.** The wound dressing of claim 1, wherein the wound dressing further comprises an active agent.

3. The wound dressing of claim 2, wherein the active agent is selected from an anti-inflammatory agent, an anesthetic, or a combination thereof.

4. The wound dressing of claim 3, wherein the active agent is a topical corticosteroid.

5. The wound dressing of claim 4, wherein the topical corticosteroid is hydrocortisone, clobetasone butyrate, or betamethasone dipropionate.

6. The wound dressing of claim 3, wherein the active agent is lidocaine or benzocaine.

7. The wound dressing of claim 1, wherein the copolymer of poly(alkylvinylether/maleic acid) is poly(methyl vinyl ether-*alt*-maleic acid) (PMVEMAC).

8. The wound dressing of claim 1, wherein the copolymer of poly(styrene/maleic anhydride) is poly(styrene-co-maleic anhydride) (SMA).

9. The wound dressing of claim 1, wherein the polymeric superporous hydrogel comprises polyvinyl alcohol (PVA).

10. The wound dressing of claim 1, wherein the average pore size of the plurality of pores ranges from about 5 $\mu$m to about 1 mm.

11. The wound dressing of claim 1, wherein the polymeric superporous hydrogel exhibits a swelling ratio between about 4000% and about 8500% after 24 hours.

12. The wound dressing of claim 1, wherein the polymeric superporous hydrogel exhibits an elastic modulus (Young's modulus) between about $1\times10^{-5}$ N/mm$^2$ and about $4\times10^{-5}$ N/mm$^2$.

13. The wound dressing of claim 1, wherein the polymeric superporous hydrogel exhibits a tensile stress at break between about $0.3\times10^{-5}$ N/mm$^2$ and about $1.3\times10^{-4}$ N/mm$^2$ .

14. The wound dressing of claim 1, wherein the polymeric superporous hydrogel exhibits a maximum strength between about $3\times10^{-2}$ N/mm and about $6.3\times10^{-2}$ N/mm.

## Patentansprüche

1. Wundauflage, umfassend ein polymeres superporöses Hydrogel, umfassend:

   ein Copolymer aus Poly(alkylvinylether/Maleinsäure), oder ein Copolymer aus Poly(styrol/Maleinsäureanhydrid);
   Polyvinylalkohol (PVA) oder Hydroxyethylcellulose (HEC); und
   eine Mehrzahl von Poren.

2. Wundauflage nach Anspruch 1, wobei die Wundauflage ferner einen Wirkstoff umfasst.

3. Wundauflage nach Anspruch 2, wobei der Wirkstoff ausgewählt ist aus einem entzündungshemmenden Wirkstoff, einem Anästhetikum oder einer Kombination davon.

4. Wundauflage nach Anspruch 3, wobei der Wirkstoff ein topisches Kortikosteroid ist.

5. Wundauflage nach Anspruch 4, wobei das topische Kortikosteroid Hydrocortison, Clobetasonbutyrat oder Betamethasondipropionat ist.

6. Wundauflage nach Anspruch 3, wobei der Wirkstoff Lidocain oder Benzocain ist.

7. Wundauflage nach Anspruch 1, wobei das Copolymer aus Poly(alkylvinylether/Maleinsäure) Poly(methylvinylether-alt-maleinsäure) (PMVEMAC) ist.

8. Wundauflage nach Anspruch 1, wobei das Copolymer Poly(styrol/Maleinsäureanhydrid) Poly(styrol-co-maleinsäureanhydrid) (SMA) ist.

9. Wundauflage nach Anspruch 1, wobei das polymere superporöse Hydrogel Polyvinylalkohol (PVA) umfasst.

10. Wundauflage nach Anspruch 1, wobei die durchschnittliche Porengröße der Mehrzahl von Poren im Bereich von etwa 5 $\mu$m bis etwa 1 mm liegt.

11. Wundauflage nach Anspruch 1, wobei das polymere superporöse Hydrogel nach 24 Stunden einen Quellungsgrad zwischen etwa 4000 % und etwa 8500 % aufweist.

12. Wundauflage nach Anspruch 1, wobei das polymere superporöse Hydrogel einen Elastizitätsmodul (Young'scher Modul) zwischen etwa $1\times10^{-5}$ N/mm$^2$ und etwa $4\times10^{-5}$ N/mm$^2$ aufweist.

13. Wundauflage nach Anspruch 1, wobei das polymere superporöse Hydrogel eine Zugspannung beim Brechen zwischen etwa $0{,}3\times10^{-5}$ N/mm$^2$ und etwa $1{,}3\times10^{-4}$ N/mm$^2$ aufweist.

14. Wundauflage nach Anspruch 1, wobei das polymere superporöse Hydrogel eine maximale Festigkeit zwischen etwa $3 \times 10^{-2}$ N/mm und etwa $6{,}3 \times 10^{-2}$ N/mm aufweist.

**Revendications**

1. Pansement de plaie comprenant un hydrogel polymère superporeux comprenant :

   un copolymère de poly(alcoylvinyléther/acide maléique), ou un copolymère de poly(styrène/anhydride maléique) ;
   de l'alcool polyvinylique (PVA) ou de l'hydroxyéthyl cellulose (HEC) ; et
   une pluralité de pores.

2. Pansement de plaie suivant la revendication 1, dans lequel le pansement de plaie comprend en outre un principe actif.

3. Pansement de plaie suivant la revendication 2, dans lequel le principe actif est choisi parmi un agent anti-inflammatoire, un anesthésique ou leurs combinaisons.

4. Pansement de plaie suivant la revendication 3, dans lequel le principe actif est un corticostéroïde topique.

5. Pansement de plaie suivant la revendication 4, dans lequel le corticostéroïde topique est l'hydrocortisone, le butyrate de clobétasone ou le dipropionate de bétaméthasone.

6. Pansement de plaie suivant la revendication 3, dans lequel le principe actif est la lidocaine ou la benzocaine.

7. Pansement de plaie suivant la revendication 1, dans lequel le copolymère de poly(alcoylvinyléther/acide maléique) est le poly(méthylvinyléther-alt-acide maléique) (PMVEMAC).

8. Pansement de plaie suivant la revendication 1, dans lequel le copolymère de poly(styrène/anhydride maléique) est le poly(styrène-co-anhydride maléique) (SMA).

9. Pansement de plaie suivant la revendication 1, dans lequel l'hydrogène polymère superporeux comprend de l'alcool polyvinylique (PVA).

10. Pansement de plaie suivant la revendication 1, dans lequel le diamètre moyen de pore de la pluralité de pores va d'environ 5 $\mu$m à environ 1 mm.

11. Pansement de plaie suivant la revendication 1, dans lequel l'hydrogel polymère superporeux a un rapport de gonflement, compris entre environ 4000% et environ 8500% après 24 heures.

12. Pansement de plaie suivant la revendication 1, dans lequel l'hydrogel polymère superporeux a un module d'élasticité

(module de Young) compris entre environ $1\times10^{-5}$ N/mm$^2$ et environ $4\times10^{-5}$ N/mm$^2$.

**13.** Pansement de plaie suivant la revendication 1, dans lequel l'hydrogel polymère superporeux a une résistance à la traction à la rupture comprise entre environ $0{,}3\times10^{-5}$ N/mm$^2$ et environ $1{,}3\times10^{-4}$ N/mm$^2$.

**14.** Pansement de plaie suivant la revendication 1, dans lequel l'hydrogel polymère superporeux a une résistance maximum comprise entre environ $3\times10^{-2}$ N/mm et environ $6{,}3\times10^{-2}$ N/mm.

FIG. 1A

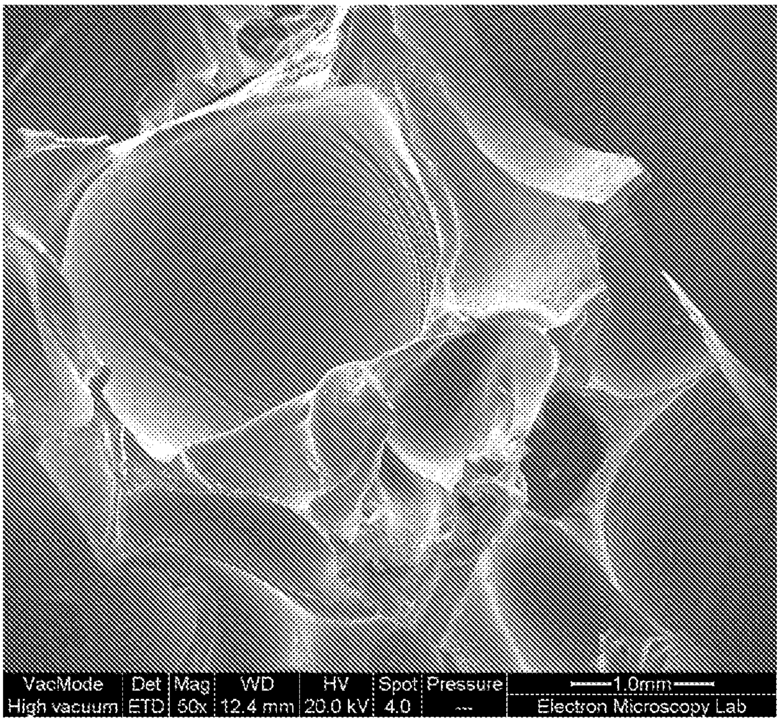
VacMode
High vacuum
Det
ETD
Mag
50x
WD
12.4 mm
HV
20.0 kV
Spot
4.0
Pressure
---
1.0mm
Electron Microscopy Lab

FIG. 1B

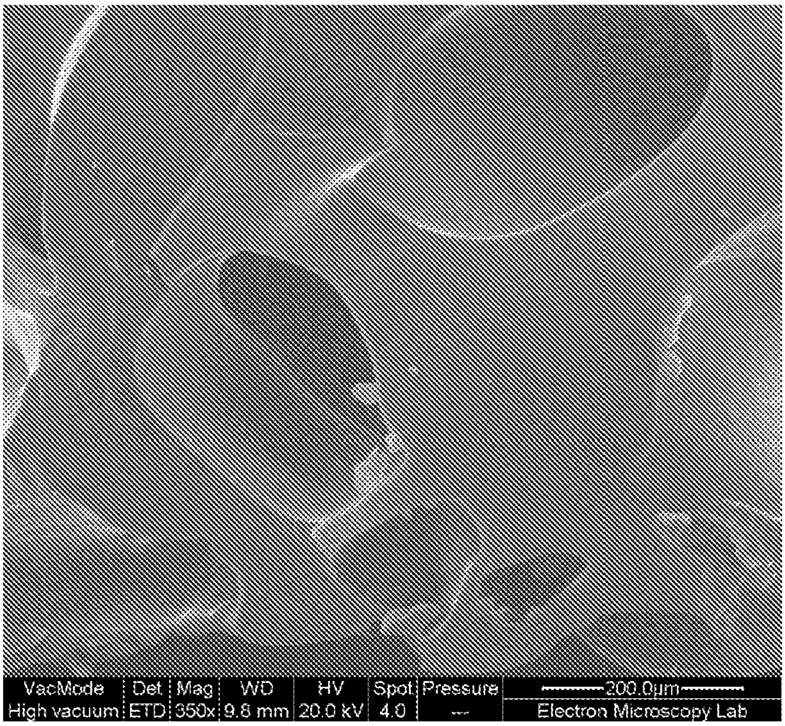
VacMode
High vacuum
Det
ETD
Mag
350x
WD
9.8 mm
HV
20.0 kV
Spot
4.0
Pressure
---
200.0µm
Electron Microscopy Lab

FIG. 2

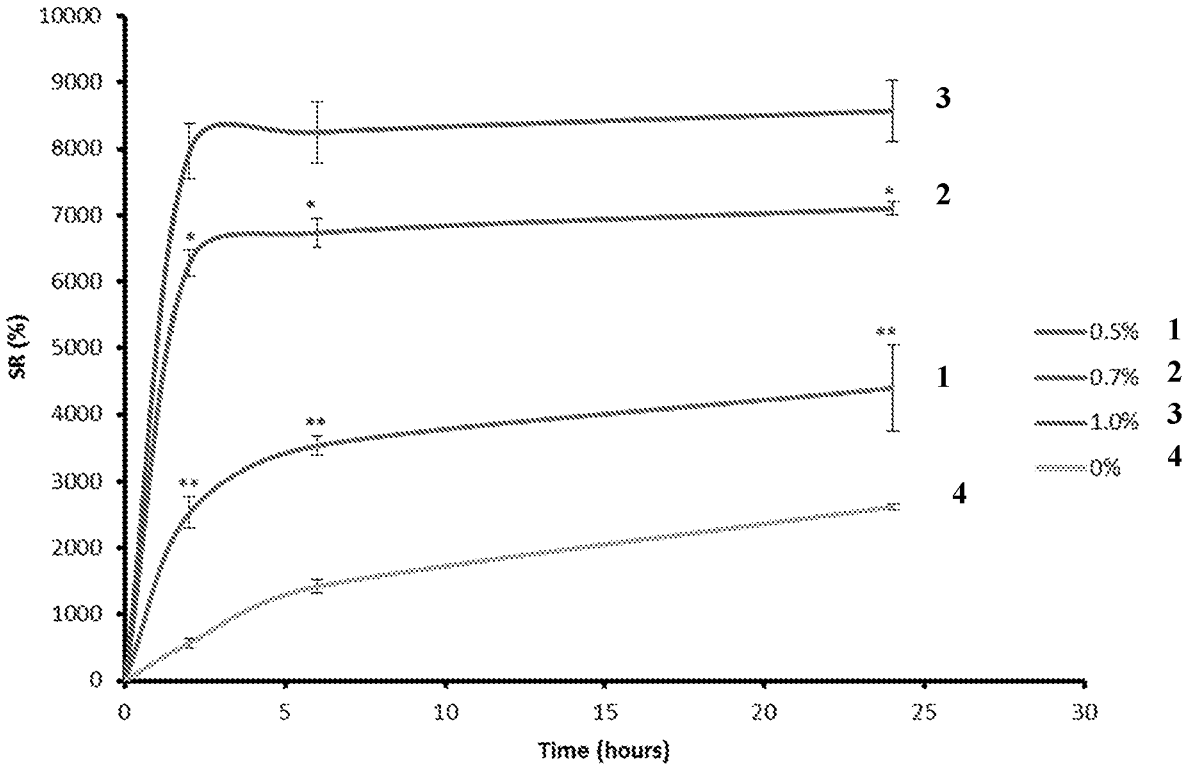
10000
9000
8000
7000
6000
5000
4000
3000
2000
1000
0
SR (%)
0
5
10
15
20
25
30
Time (hours)
3
2
1
4
*
**
0.5% 1
0.7% 2
1.0% 3
0% 4

FIG. 3

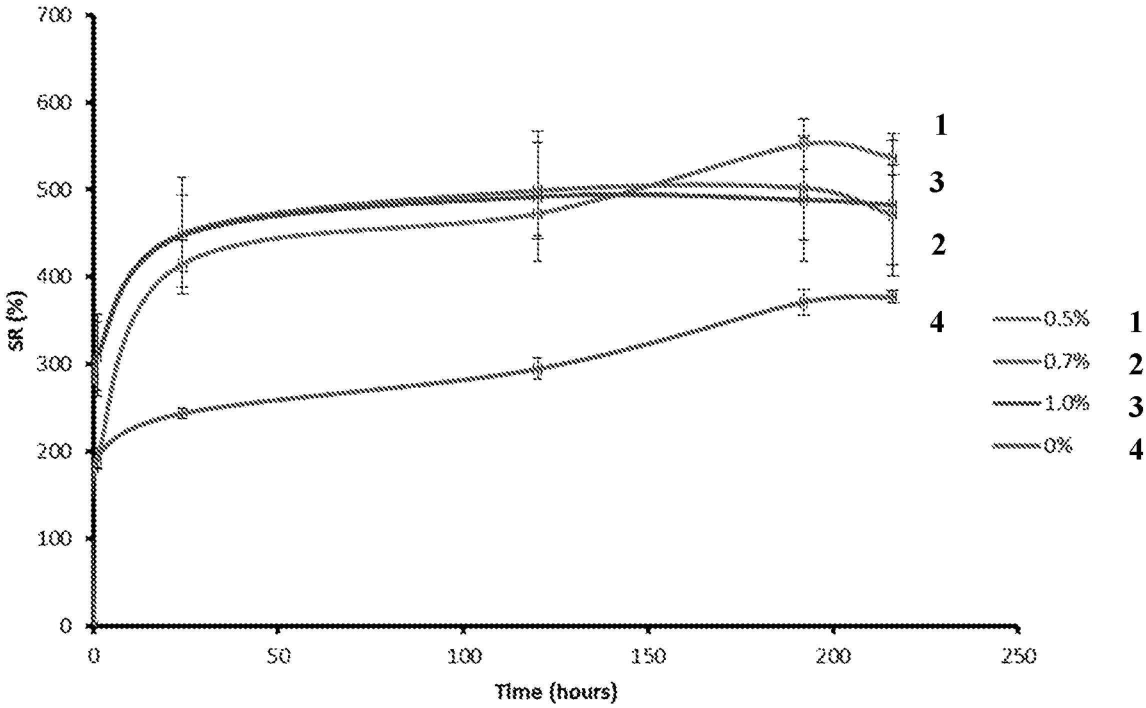
700
600
500
400
300
200
100
0
SR (%)
0
50
100
150
200
250
Time (hours)
1
3
2
4
0.5% 1
0.7% 2
1.0% 3
0% 4

**FIG. 4A**

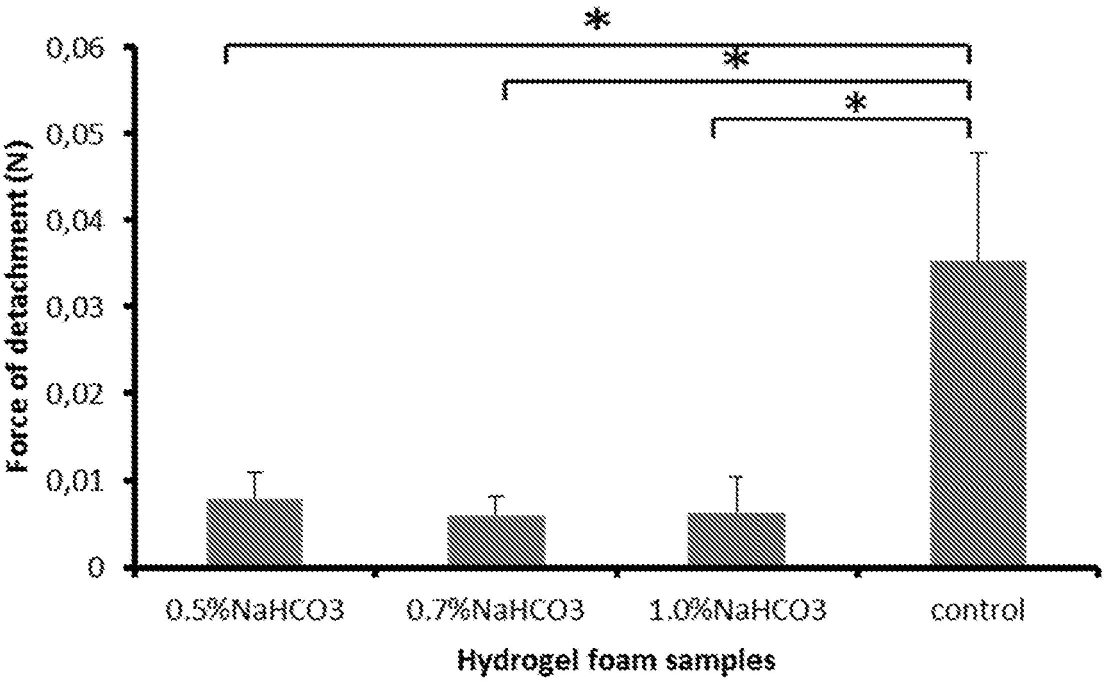
*
*
*
0,06
0,05
0,04
0,03
0,02
0,01
0
Force of detachment (N)
0.5%NaHCO3
0.7%NaHCO3
1.0%NaHCO3
control
Hydrogel foam samples

**FIG. 4B**

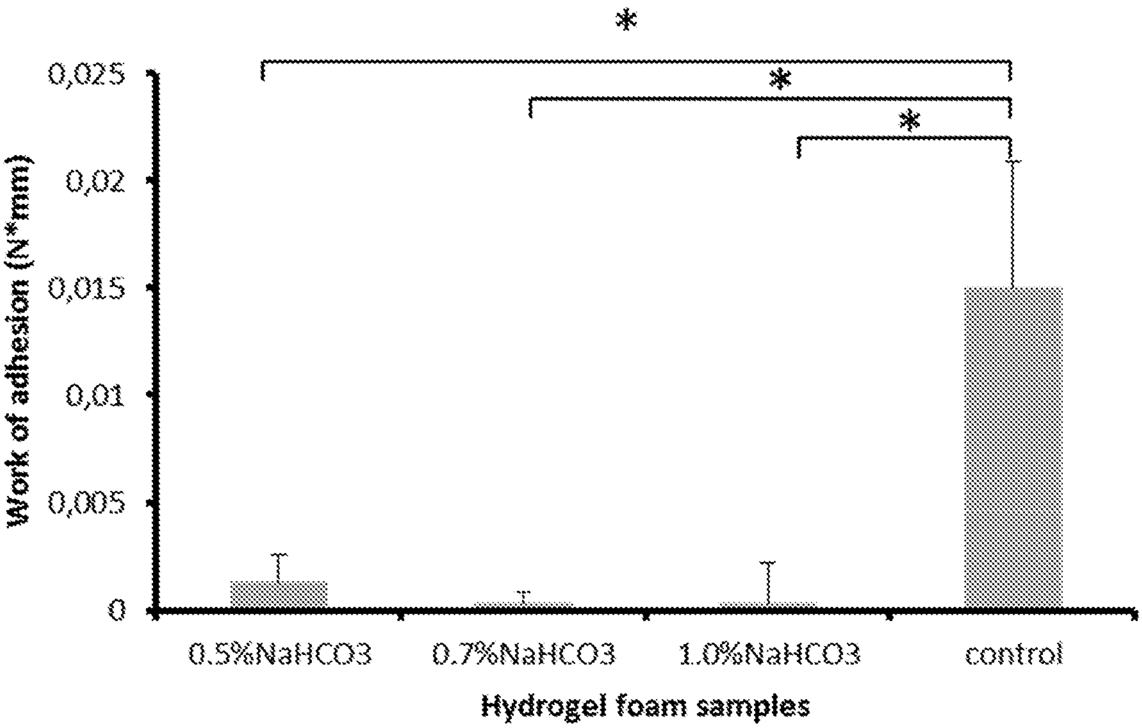
*
*
*
0,025
0,02
0,015
0,01
0,005
0
Work of adhesion (N*mm)
0.5%NaHCO3
0.7%NaHCO3
1.0%NaHCO3
control
Hydrogel foam samples

FIG. 4C

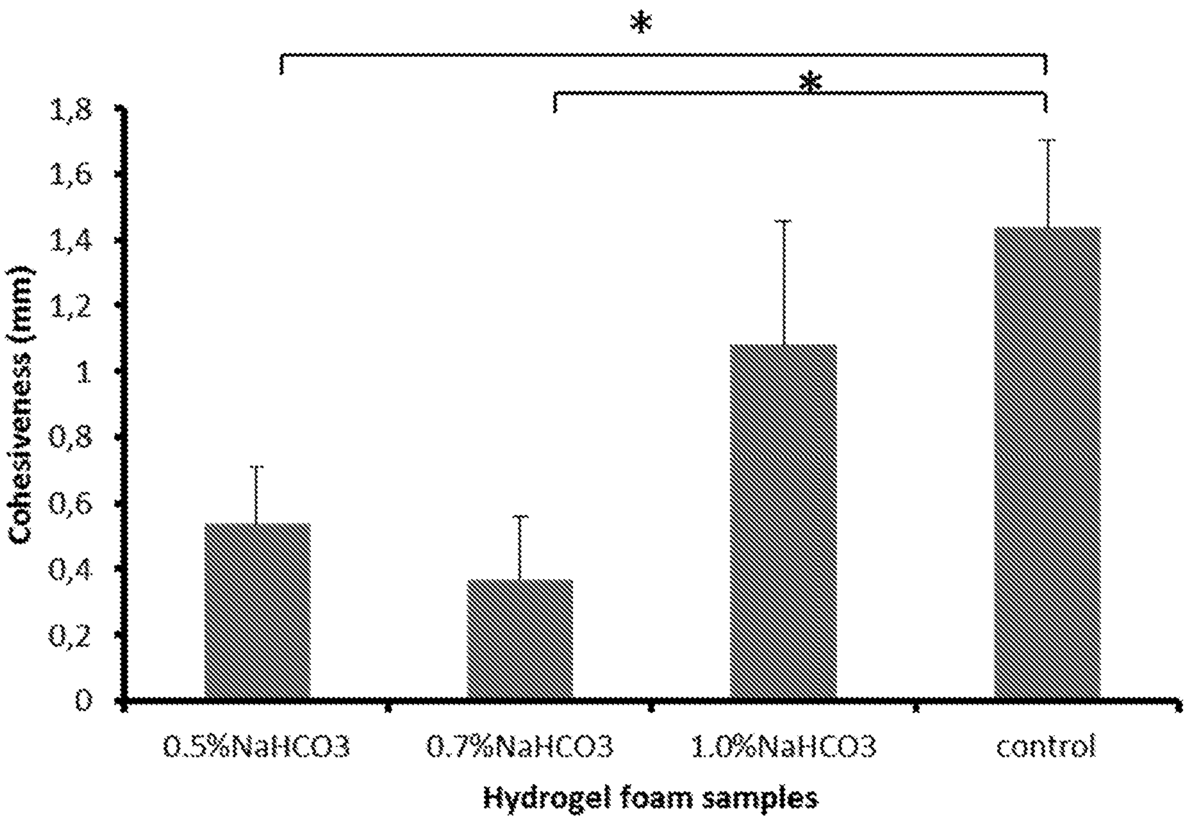
*
*
Cohesiveness (mm)
1,8
1,6
1,4
1,2
1
0,8
0,6
0,4
0,2
0
0.5%NaHCO3
0.7%NaHCO3
1.0%NaHCO3
control
Hydrogel foam samples

FIG. 5A

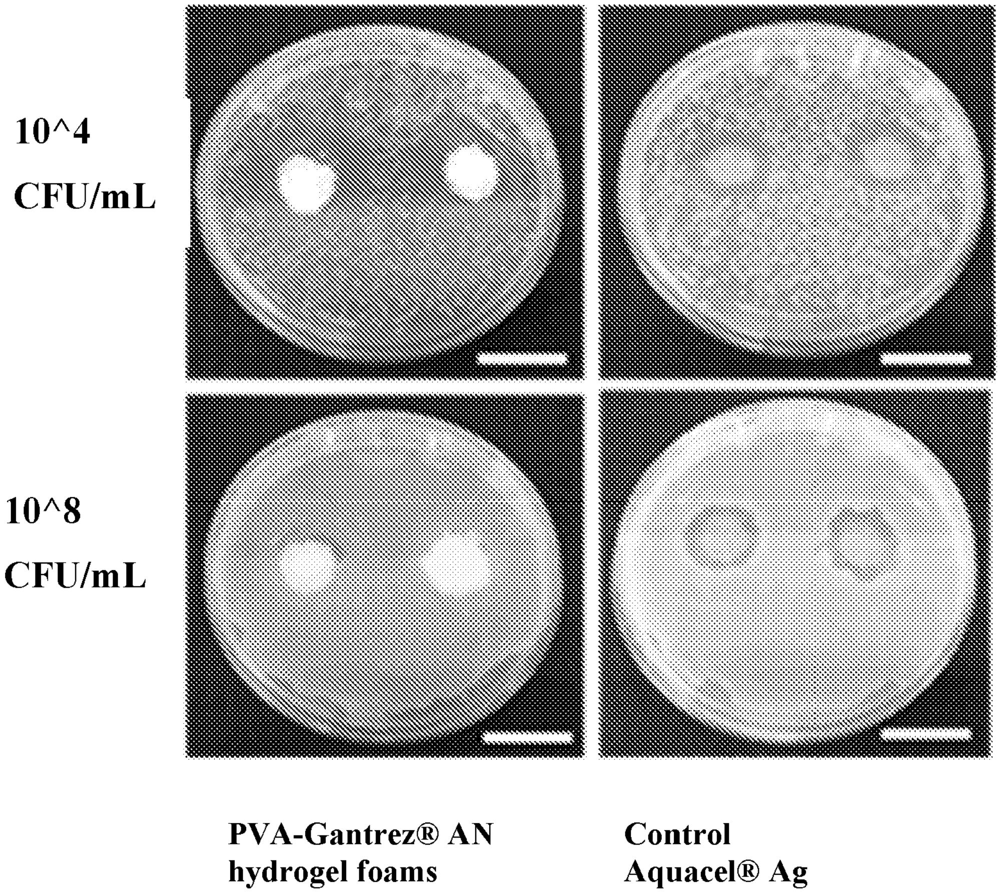
10^4
CFU/mL
10^8
CFU/mL
PVA-Gantrez® AN
hydrogel foams
Control
Aquacel® Ag

FIG. 5B

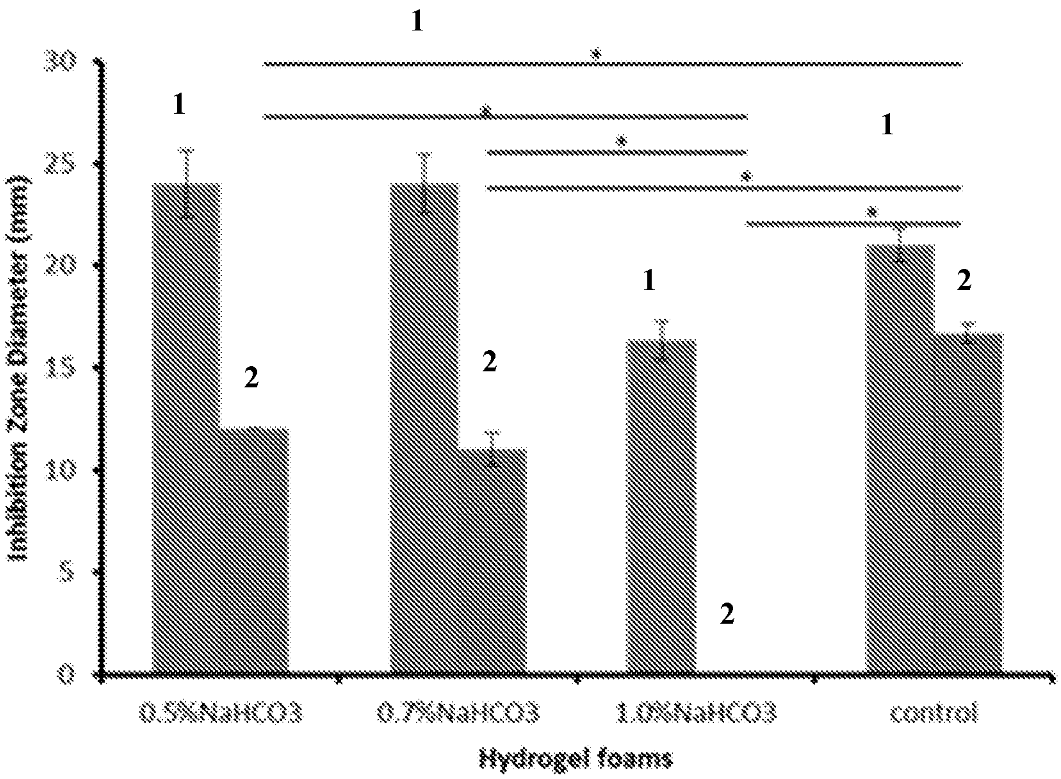


1: 10^4 CFU/mL

2: 10^8 CFU/mL

FIG. 6

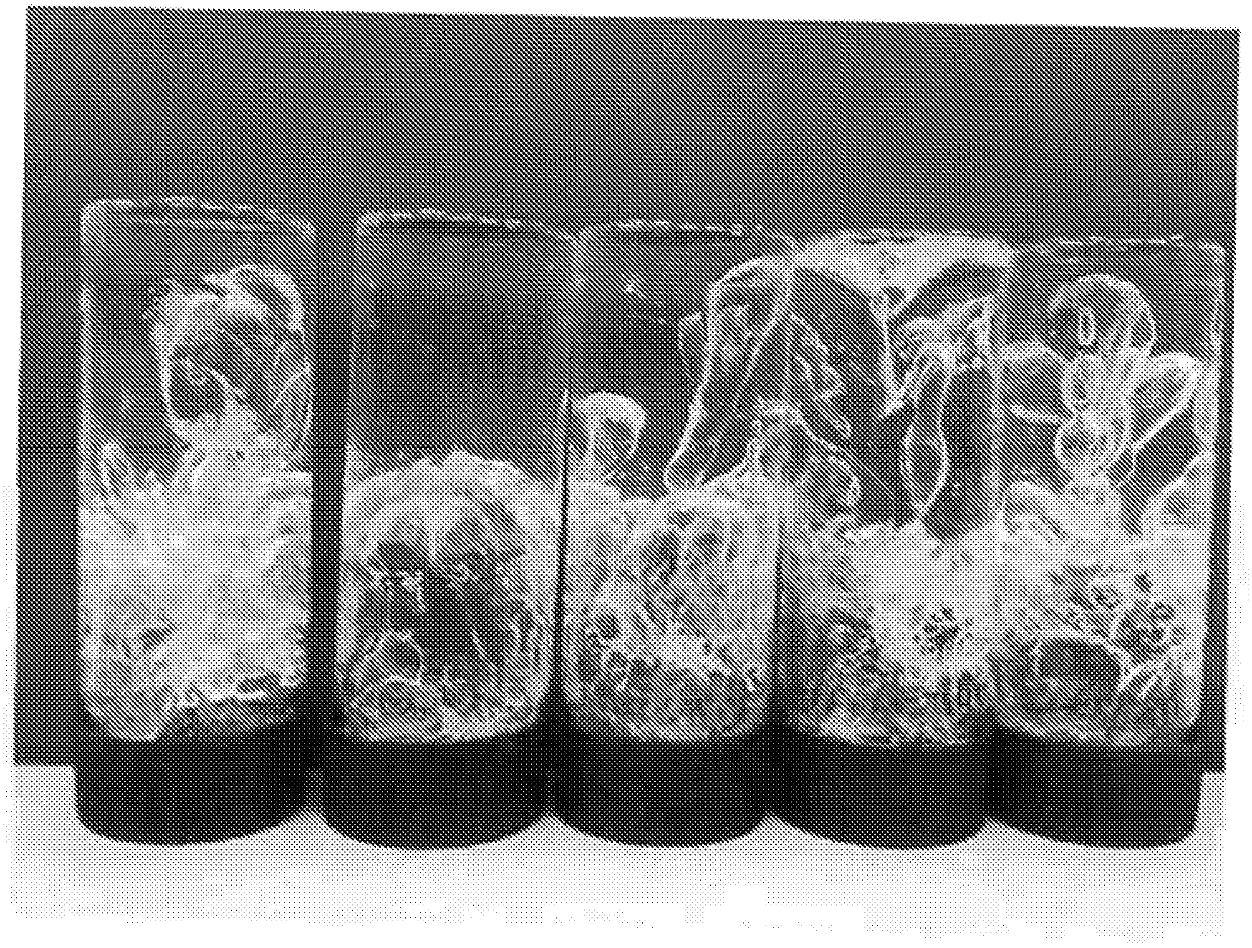

(a) (b) (c) (d) (e)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62832777
- US 6271278 B


### Non-patent literature cited in the description


- **CHEN et al.** *J. Biomed. Mater. Res.,* 1999, vol. 44, 53-62
- **CHEN J. et al.** Synthesis and Characterization of Superporous Hydrogel Composites. *I Control. Release,* 2000, vol. 65 (1-2), 73-82
- **OMIDIAN H. et al.** Advances in Superporous Hydrogels. *I Control. Release,* 2005, vol. 102 (1), 3-12
- **DRAGAN E. S.** Design and Applications of Interpenetrating Polymer Network Hydrogels. A Review. *Chem. Eng. 1,* 2014, vol. 243, 572-590
- **CALÒ E. et al.** Antimicrobial Hydrogels Based on Autoclaved Poly(vinyl Alcohol) and Poly(methyl Vinyl Ether-Alt- Maleic Anhydride) Mixtures for Wound Care Applications. *RSC Adv.,* 2016, vol. 6, 55211-55219
- **HALLANDER H. 0. et al.** Identification of Cephalosporin-Resistant Staphylococcus Aureus with the Disc Diffusion Method. *Antimicrob. Agents Chemother,* 1972, vol. 1 (5), 422-426
- **BOONKAEW B. et al.** Antimicrobial Efficacy of a Novel Silver Hydrogel Dressing Compared to Two Common Silver Burn Wound Dressings: Acticoat' and PolyMem Silver. *Burns,* 2014, vol. 40 (1), 89-96